# EUROPÄISCHE PATENTANMELDUNG

(11) **EP 0 933 078 A2**
(43) Veröffentlichungstag der Anmeldung: **04.08.1999**
(21) Anmeldenummer: 98122229.2
(22) Anmeldetag: 23.11.1998
(51) Int. Cl.: A61K 7/16, A61K 7/18

(54) **Wirkstoff enthaltende Fällungskieselsäuren**

(30) Priorität: 10.12.1997 DE 19754798; 28.01.1998 DE 19803066
(71) Anmelder: Degussa-Hüls Aktiengesellschaft, 45764 Marl (DE)
(72) Erfinder: Esch, Heinz, 53117 Bonn (DE); Kuhlmann, Robert, 50374 Erfstadt (DE); Neumüller, Matthias, 63594 Hasselroth (DE); Otto, Karin Dr., 53121 Bonn (DE); Rausch, Ralf Dr., 52372 Kreuzau (DE); Thomas, Klaus-Peter, 61200 Wölfersheim (DE)

(57) **Zusammenfassung**

Wirkstoff enthaltende Fällungskieselsäure mit einer charakteristischen, verzögerten Wirkstofffreigabe wird hergestellt, indem man bei der Herstellung der Fällungskieselsäure nach einem bekannten Verfahren den Wirkstoff hinzufügt.

Die Fällungskieselsäure kann Mundpflegemitteln, wie Zahnpasten, topischen Fluoridpräparationen, Dentalwerkstoffen oder medizinischen Kaugummis zugesetzt werden.

## Beschreibung

Die Erfindung betrifft Wirkstoff enthaltende Fällungskieselsäuren, deren Herstellung und Verwendung in Mundpflegemitteln.

Fluorid-Ionen spielen bei der Kariesprophylaxe eine entscheidende Rolle. So werden unter anderem Fluorid-Ionen dem Trinkwasser zugesetzt, um der Weiterverbreitung von Karies zu begegnen.

Eine andere Form des Einsatzes von Fluorid-Ionen zur Bekämpfung von Karies ist die Fluorid-Ionen enthaltende Zahnpasta (vgl. 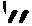 Oral Hygiene Products and Practice" von Morton Pader in 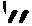 Cosmetic Science and Technology Series", Vol. 6, Seiten 383 ff., (1988), Marcel Dekker, Inc.). Hierzu ist es bekannt, Zahnpasten Fluorid-Ionen in Form von NaF, MFP, Aminfluorid, Zinnfluorid etc., zuzusetzen. Daneben können diese Zahnpasten Fällungskieselsäuren, die eine Abrasivwirkung und/oder Verdickungswirkung aufweisen, enthalten (WO 93/24103). Die Verfügbarkeit der Fluorid-Ionen in der Mundhöhle beschränkt sich jedoch im wesentlichen auf die kurze Dauer des Zähneputzens.

Es ist bekannt, zu einer wäßrigen Aufschlämmung von Kieselgel bzw. Xerogel Fluorwasserstoffsäure hinzuzugeben. Diese Aufschlämmung wird anschließend mit den Komponenten zur Herstellung einer Zahnpasta vermischt, wobei unter anderem auch Calciumchlorid hinzugegeben wird (DE-A 24 16 742). Die Behandlung mit Flußsäure bewirkt, daß Si-F-Bindungen auf dem Kieselgel ausgebildet werden una dadurch Kationen, wie zum Beispiel Calciumionen, weniger von dem Kieselgel adsorbiert werden. Die Bildung von Fluoranionen und deren kontrollierte Freisetzung in der Zahnpasta wird in dem Dokument DE-A 24 16 742 nicht beschrieben.

Es ist bekannt, pyrogen hergestelltes Silicumdioxid, welches 1 bis 5 % Fluorid adsorbiert enthält, in Zahnpasten einzuarbeiten. Diese Zahnpasta enthält stets 800 bis 1200 ppm freies Fluorid, welches für eine entsprechende therapeutische Behandlung zur Verfügung steht (US-A 4 172 121). Das pyrogen hergestellte Siliciumdioxid wirkt in dieser Zahnpasta neben seiner Eigenschaft als Fluorid-Ionen enthaltendes Medium auch als Verdickungsmittel, um der Zahnpasta die gewünschte pastenförmige Konsistenz zu geben.

Die Fluorid enthaltende pyrogene Kieselsäure wird gemäß der US-A 4 054 689 durch Behandeln der pyrogenen Kieselsäure mit HF-Gas hergestellt.

Es ist bekannt, eine Fluorid enthaltende Fällungskieselsäure herzustellen, indem man in eine wäßrige Vorlage Natriumfluorid und Oxalsäure gibt und anschließend durch gleichzeitige Zugabe von Natriumsilikatlösung und Schwefelsäure die Fällungskieselsäure ausfällt (DE-B 12 93 138).

Aufgrund des Zusatzes von Natriumfluorid und Oxalsäure werden Eisenionen in der Fällmischung komplexiert und eine Fällungskieselsäure mit einem niedrigen Eisengehalt erhalten. Eine Adsorption von Natriumfluorid an der Fällungskieselsäure findet nicht statt.

Ein entscheidender Faktor für die Verbesserung der Wirksamkeit von Mundpflegepräparaten wird vor allem darin gesehen, daß nach einer spontanen Freisetzung der Wirkstoffe die Wirkstoffkonzentration im Mundraum möglichst lange erhalten bleibt. Ziel ist es, die Wirkstoffretention zu verbessern, und damit die gewünschte Wirkung auch mit reduzierten Wirkstoffdosen zu erzielen. Auf diese Weise sollen unerwünschte Nebenwirkungen durch hohe Dosierungen weitgehend vermieden werden.

Am Beispiel des Wirkstoffs Fluorid, dessen Antikarieswirkung in zahlreichen klinischen und epidemiologischen Studien nachgewiesen wurde, konnte gezeigt werden, daß der gewünschte Kariesschutz schon mit äußerst geringen Konzentrationen erzielt wird (siehe Y. Ericson, J.Dent.Res. 59 (DII):2131 (1980); O. Backer Dirks, W. Künzel, and J.P. Carlos, Caries Res. 12 (Suppl.1):7 (1978); A.r. Volpe, in a textbook of Preventive Dentistry (R.C. Caldwell and R.E. Stallard, eds.), Saunders, Philadelphia, 1997, Chap. 12).

Ferner gibt es Hinweise, daß die Häufigkeit der Fluorid-Applikation, d. h. die Anwesenheit von Fluoridionen zur richtigen Zeit am richtigen Ort, bei einem Säureangriff von größerer Bedeutung als die Fluoridkonzentration ist (siehe 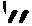 Oral Hygiene Products and Practice" von Morton Pader in 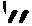 Cosmetic Science and Technology Series" Vol. 6, Seiten 383 ff., (1988), Marcel Dekker, Inc.).

Diese Erkenntnisse führten zu der Forderung, daß Forschungsaktivitäten darauf ausgerichtet werden sollten, die Dauer der Fluorideinwirkung bei gleichzeitiger Minimierung der Fluoridkonzentration zu maximieren. Auch toxikologische Aspekte dürfen bei Erhöhung der Fluoriddosen oder Akkumulierungen, z. B. durch fluoriertes Trinkwasser, Mundwässer, Zahnpasten, topische Fluoridpräparationen, Fluoridtabletten etc., nicht außer Betracht gelassen werden.

Auch im Fall von Anti-Plaque-Wirkstoffen konnte durch in vitro- und in vivo-Studien gezeigt werden, daß der Schlüssel zu einer verbesserten Wirkung in einer verbesserten Wirkstoffretention zu suchen ist. So wird beispielsweise eine außerordentliche Plaqueinhibierung durch Chlorhexidin erzielt, was hauptsächlich auf dessen Retention im Mundraum und allmählicher Freigabe aus der Plaquematrix und den Schleimhäuten zurückgeführt wird. Eine lang anhaltende antimikrobielle Wirkung wurde in frühen Studien durch Keimzahlbestimmungen im Speichel nach einmaligen Spülungen nachgewiesen (siehe K. Kornmann: Antimicrobial agents. In Loe, H & Kleinmann DV: Dental Plaque control measures and oral hygiene practices, Oxford / Washington, IRL Press, 121-142, 1986; P. Germo, P. Bonesvoll, G. Rolla: Relationship between plaque inhibiting effect and retention of Chlorhexidine in the oral cavity. Archs Oral Biol. 19:1031-1034, 1974; W.R. Roberts, M. Addy: Comparison of the bisguanidine antiseptics, alexine and chlorhexidine: i Effects on plaque accumulation and salivary bacteria. J.Clin.Periodontol. 8:8 213-219, 1981).

Ein weiteres Beispiel bietet Triclosan (2,4,4'-Trichloro-2'-hydroxydiphenylether), das erst in Kombination mit einem Copolymer (Polyvinylmethylether/Maleinsäure), welches die Retention von Triclosan verbessert, eine deutliche Plaqueinhibierung aufweist (siehe American Journal of Dentistry, Vol. 2, Special Issue, September, 1989: Report on the use of Triclosan/Copolymer Dentifrices in the Control of Plaque and Gingivitis).

Es ist bekannt, daß Kieselsäuren als hochdisperse Stoffe in der Lage sind, sich in Fissuren, Mikrorissen und Tubuli von Zahnoberflächen einzulagern. Die Blockierung freiliegender Dentintubuli durch Kieselsäure führt auf diese Weise zu einem desensibilisierenden Effekt (siehe M. Addy, P. Mostafa und R. Newcombe, Dentine Hypersensitivity: A Comparison of five toothpastes used during a six-week treatment period; British Dent. J. 163, 45-50, 1987).

Es besteht somit die Aufgabe, eine Fällungskieselsäure mit Wirkstoffträgerfunktion für Mundpflegemittel herzustellen, die alle an derartige Mittel gestellten Anforderungen hinsichtlich Verträglichkeit, Abrasivität, Rheologie, organoleptischen und optischen Eigenschaften erfüllt, und in der Lage ist, Wirkstoffe im Mundraum zu fixieren und kontrolliert über längere Zeiträume wieder abzugeben.

Gegenstand der Erfindung sind Wirkstoff enthaltende Fällungskieselsäuren, welche gekennzeichnet sind durch eine charakteristische, verzögerte Wirkstofffreigabe.

Vorzugsweise kann die Wirkstoff enthaltende Fällungskieselsäure gekennzeichnet sein durch:
- den Wirkstoffgehalt
- die Wirkstoff-Freigabedynamik

In einer bevorzugten Ausführungsform kann die erfindungsgemäße Fällungskieselsäure folgende physikalischchemische Parameter

| | | | |
|---|---|---|---|
| Feuchte | % | 1 - 10, | vorzugsweise 1 - 7 |
| pH | | 2,5 - 8,5, | vorzugsweise 6 - 8 |
| N₂-Oberfläche | m²/g | 25 - 800, | vorzugsweise 25 - 400 |
| Mittlere Teilchengröße Malvern | µm | 2 - 100, | vorzugsweise 2 - 20 |

aufweisen.

Ein weiterer Gegenstand der Erfindung ist ein Verfahren zur Herstellung der erfindungsgemäßen Fällungskieselsäure mit Wirkstoff-Trägerfunktion, welches dadurch gekennzeichnet ist, daß man eine Fällungskieselsäure auf bekanntem Wege herstellt, wobei ein in Wasser mäßig bis schwer löslicher Wirkstoff entweder in die Fällungsvorlage oder in die Fällungssuspension gegeben oder zusammen mit dem gewaschenen und gegebenenfalls redispergierten Fällungskieselsäureteig getrocknet oder gleichzeitig mit dem getrockneten Material vermahlen wird.

Die Herstellung amorpher Kieselsäuren ist in mannigfacher Weise beschrieben. Allen Verfahren gemeinsam ist die Umsetzung von Alkalimetall-Silikat-Lösung mit Mineralsäure oder CO₂ unter Einhaltung bestimmter Fällbedingungen, wie z. B. Temperatur, Zeit, pH-Wert, Feststoffgehalt in der Fällsuspension. Anschließend kann der pH-Wert durch weitere Säurezugabe abgesenkt werden. Während der Fällungs- und Säuerungsphase wird ständig gerührt. Die Suspension wird filtriert, der Kuchen ausgewaschen, getrocknet und vermahlen.

Die erfindungsgemäßen amorphen Kieselsäuren werden in der Weise hergestellt, daß man eine Fällungskieselsäure auf bekanntem Wege herstellt, wobei ein in Wasser mäßig (0,1 - 1,0 molar) bis schwer löslicher (weniger als 0,1 molar) Wirkstoff entweder in die Fällungsvorlage oder in die Fällungssuspension gegeben oder zusammen mit dem gewaschenen und gegebenenfalls redispergierten Kieselsäureteig getrocknet oder gleichzeitig mit dem getrockneten Material vermahlen wird.

Als fluoridionenhaltige Komponente können z. B. Alkali-, Erdalkalifluoride oder Fluorapatit eingesetzt werden, wobei Fluoride aus Alkaliverbindungen zusätzlich durch Zugabe von Erdalkaliionen gebunden werden können. Weitere Wirkstoffe können sein: Chlorhexidine, Triclosan, Hydroxyäthan-1,1-diphosphonsäure, Alkalipyrophosphate, Zinkcitrat und etc.

Die Herstellung der Fällungskieselsäure kann erfolgen, wie sie in den Dokumenten DE-A 44 23 493, DE-AS 14 67 019 und EP-B 0 272 380 beschrieben wird.

Es hat sich nun gezeigt, daß eine bevorzugte Form der Herstellung darin besteht, daß man den Wirkstoff in die Fällungsvorlage beziehungsweise die Sprühtrocknerspeise gibt. Hierdurch ist die gleichmäßigste und feinste Verteilung im Fertigprodukt und die größte Retentionswirkung gewährleistet. Die therapeutisch wirksamen Fluorid-Mengen betragen 0,001 bis 10 %, vorzugsweise 0,005 bis 1,0 %. Die Triclosan-Mengen betragen 0,1 - 1,0 %, vorzugsweise 0,2 - 0,5 %.

Als Trägerkieselsäuren kommen generell alle Verdickungs-, Abrasiv- oder bifunktionelle Kieselsäuren in Frage. Bevorzugt sind synthetische amorphe Kieselsäuren, wie sie in EP 0 643 015 beschrieben und von der Fa. Degussa AG unter der Markenbezeichnung Sident® erhältlich sind. Ebenso können Zeodent-Kieselsäuren der Fa. J.M. Huber Corporation, Chemical Division, Havre de Grace, Maryland, Tixosil-Kieselsäuren der Fa. Rhone-Poulenc Chimie Les Miroirs- La defense 3 F-92400 Courbevoie, Sorbosil-Kieselsäuren der Fa. Crosfield Chemicals, Warrington Cheshire, England oder Syloid-Kieselsäuren der Fa. Grace & Co., Davison Chem. Division, eingesetzt werden.

Bevorzugt kann die Herstellung der erfindungsgemäßen Fällungskieseläure mit Wirkstoffträgerfunktion erfolgen, indem man in einem Fällgefäß Wasser und den Wirkstoff vorlegt, die Mischung auf eine Temperatur von 50 bis 100 °C, vorzugsweise 80 bis 90 °C erhitzt, unter Aufrechterhaltung der Temperatur mittels Zugabe von Natriumsilikat-Lösung den pH-Wert bzw. den Alkaligehalt der Mischung einstellt, anschließend gleichzeitig unter Konstanthalten des pH-Wertes bzw. des Alkaligehaltes oder nacheinander Natriumsilikat-Lösung und Schwefelsäure hinzugibt, danach mittels weiterer Schwefelsäurezugabe vorzugsweise auf einen pH-Wert von 7, ansäuert, gegebenenfalls nachrührt, abfiltriert, salzfrei wascht, den Filterkuchen redispergiert, mittels eines Sprühtrockners trocknet und das erhaltene Kieselsäuregranulat vermahlt.

Die handelsübliche Natriumsilikatlösung kann z. B. eine Konzentration von 26,8 % SiO₂ und 7,85 % Na₂O bei einer Dichte 1,352 g/ml aufweisen.

Die Schwefelsäure kann eine Konzentration von 50 - 96 % aufweisen.

Die erfindungsgemäßen Kieselsäuren können in üblicher, dem Fachmann bekannter Weise in Mundpflegepräparaten wie Zahnpasten, medizinischen Kaugummis und topischen Fluoridpräparationen sowie Dentalwerkstoffen zum Einsatz kommen. Dabei ersetzen sie die dort üblicherweise verwendeten Kieselsäuren ganz oder teilweise.

Bevorzugtes Einsatzgebiet ist das der Zahnpasten. Darüber hinaus ist aber auch eine Kombination mit anderen, ggf. löslichen Wirkstoffen sowie die Herstellung von Gelformulierungen etc., möglich.

Erfindungsgemäße Zahnpflegemittel können eine oder mehrere der nach dem Stand der Technik verwandten Rohstoffe, wie Wasser, Bindemittel, wie z. B. Cellulosederivate wie Carboxymethylcellulosen und deren Alkalisalze, insbesondere des Natriums, Hydroxyalkylcellulosen, Xanthangum, Tragant, Carragenate, Alginate und Gummiarabicum etc., Putzkörper (gefällte und pyrogen hergestellte Kieselsäuren, Dicalciumphosphat, Kreide, Aluminiumhydroxid, etc.), Feuchthaltemittel wie z. B. Glyzerin, Sorbitol, Propylenglykol, Polyethylenglykole niederen Molekulargewichtes, 1,4 Butandiol, Xylit etc., Geschmackstoffe, Tenside wie z. B. Alkylsulfate, Natriumlaurylsulfat, Sarcoside, Taurinfettsäureamide Monoglyzeridsulfat, Betaine etc., Farbstoffe und/oder Titandioxid, Süßstoffe, Puffer, Basen oder Säuren, Konservierungsmittel und Wirkstoffe enthalten.

Die Herstellung der Zahnpflegemittel erfolgt gemäß Stand der Technik. Die Inhaltsstoffe werden dabei in geeigneter Form miteinander gemischt, gequollen und dispergiert.

Die erfindungsgemäße Fällungskieselsäure kann als Zusatz, insbesondere als Verdickungs- und/oder Abrasivkomponente, die zusätzlich Wirkstoffe abgibt, in Zahnpasten eingesetzt werden.

Die erfindungsgemäße Fällungskieselsäure weist die folgenden Vorteile auf:

Die erfindungsgemäßen Kieselsäuren dienen als Träger für zahnmedizinische Wirkstoffe, die sich am Wirkort einlagern und die Wirkstoffe in geringen Dosen über längere Zeiträume wieder abgeben (Depotwirkung, controlled release). Die Kieselsäuren wirken somit als Wirkstoffspeicher, die die Wirkstoffe in adsorbierter, absorbierter oder chemisorbierter Form enthalten. Es können alle Formen von Kieselsäuren, z. B. Fällungskieselsäuren oder Kieselgele, pyrogene Kieselsäuren, verwendet werden. Als Wirkstoffe dienen alle schwerlöslichen Fluoride, wie z. B. CaF₂ sowie schwerlösliche Wirkstoffe, wie z. B. Triclosan oder Chlorhexidin.

Die schwerlöslichen Wirkstoffe können mit leicht löslichen Wirkstoffen, wie z. B. NaF, Monofluorphosphat etc. kombiniert werden, wodurch eine gezielte Einstellung der Wirkstoff-Freigabe-Dynamik, das heißt, eine gezielte Kombination von Sofort- und Depotwirkung möglich ist. Die Vorteile der erfindungsgemäßen Kieselsäuren liegen in der längeren Verfügbarkeit und damit verbesserten therapeutischen Wirksamkeit z. B. verbesserter Kariesprophylaxe durch Verbesserung der Härteresistenz gegenüber Demineralisierung. Dadurch sind geringe Wirkstoffdosen möglich und weniger Nebenwirkungen bzw. geringere Toxizität zu erwarten. Es kann auf den Zusatz von Fluorid zum Trinkwasser, d. h. eine Zwangsmedikamentierung, verzichtet werden.

### Beispiele

Mögliche Varianten für die Herstellung der erfindungsgemäßen Fällungskieselsäure werden am Beispiel der Fluoriddotierung erläutert:
*Herstellungsvariante 1*
   Fluoridsalze in die Fällungsvorlage + Erdalkalichloride am Fällungsende
*Herstellungsvariante 2*
   Fluoridsalze + Erdalkalichloride am Ende der Fällung
*Herstellungsvariante 3*
   Fluoridsalze in der Fällungsvorlage
*Herstellungsvariante 4*
   Fluoridsalze in die Sprühtrocknerspeise
*Herstellung der Fluorid-Ionen enthaltenden abrasiven Fällungskieselsäure*

### Beispiel 1 (Fluoridhaltige Abrasivkieselsäure)

Die Fällung wird im wesentlichen gemäß DE-A 44 23 493, Beispiel 1 unter Zusatz von CaF₂ in die Fällungsvorlage durchgeführt.

In einem 50 l-Fällgefäß mit indirekter Beheizung werden unter Rühren 12,8 l Wasser und 52,8 g CaF₂ vorgelegt und auf 85 °c aufgeheizt. Unter Aufrechterhaltung dieser Temperatur wird zunächst durch Zugabe von wenig Natriumsilikatlösung (26,8 % SiO₂ und 7,85 % Na₂O, Dichte 1,352 g/ml) ein pH-Wert von 8,5 eingestellt. Danach wird durch Zugabe von 60,0 ml/min Wasserglas (Zusammensetzung wie zuvor angegeben) und soviel Schwefelsäure (50 %ig), daß pH 8,5 konstant eingehalten wird, 240 Minuten lang gefällt.

Die erhaltene Suspension wird anschließend mit Schwefelsäure (50 %ig) bis ≦ pH 7 gesäuert.

Die Reaktionsmischung wird 60 Minuten nachgerührt, abfiltriert, salzfrei gewaschen und in einem Sprühtrockner getrocknet. Das Produkt wird anschließend vermahlen.

Die erhaltene Fällungskieselsäure weist die folgenden physikalisch-chemischen Kenndaten auf:

| | | |
|---|---|---|
| Feuchte | % | 3,2 |
| pH-Wert | | 8,0 |
| Leitfähigkeit | µS/cm | 400 |
| N₂-Oberfläche | m²/g | 31 |
| Mittlere Teilchengröße (Malvern) | µm | 10,2 |
| Fluorid-Ion-Gehalt | % | 0,5 |

### Beispiel 2 (Vergleichsbeispiel fluoridfreie Abrasivkieselsäure)

Es wird eine Abrasiv-Fällungskieselsäure ohne Fluoridzusatz gemäß DE-A 44 23 493, Beispiel 1, hergestellt.

### Beispiel 3 (fluoridhaltige Fällungskieselsäure mit Verdickungswirkung)

Das Verfahren wird gemäß EP 0 272 380 B1, Beispiel 1, durchgeführt.

In einem gummierten 120 l-Fällgefäß werden 73 l heißes Wasser und 5,25 l Natriumsilikatlösung (Dichte 1,353 g/ml, Modul SiO₂ : Na₂O = 3,46) unter Rühren auf 85 °C erhitzt. In diese alkalische Fällungsvorlage werden während der folgenden 90 Minuten unter Rühren und Aufrechterhaltung der Temperatur gleichzeitig 16,5 l Natriumsilikatlösung (Zusammensetzung wie oben) und 1,448 l Schwefelsäure (96 %ig) zudosiert.

Danach wird die so erhaltende Fällungskieselsäuresuspension mit Schwefelsäure (96 %ig) auf einen pH-Wert von 3,5 eingestellt, was durch einen mehrere Minuten lang andauernden Säurezufluß mit 1,25 l/Stunde geschieht. Die auf diese Weise erhaltene Fällungskieselsäuresuspension hat einen Feststoffgehalt von 85,0 g/l.

Der nach dem Abfiltrieren und Auswaschen erhaltene restsalzarme Teig wird unter intensiver Rührung und Zusatz von Wasser sowie soviel CaF₂, daß der F-Gehalt bezogen auf die Kieselsäuremenge 0,5 Gewichts-% beträgt, in eine sprühfähige Suspension überführt, sprühgetrocknet und auf der Lufstrahlmühle vermahlen.

### Beispiel 4 (Vergleichsbeispiel fluoridfreie Fällungskieselsäure mit Verdickungswirkung)

Das Verfahren wird gemäß EP 0 272 380 B1, Beispiel 1, durchgeführt, wobei kein CaF₂ zugesetzt wird.

### Zahnpasta-Rezepturen, bei denen die Fällungskieselsäuren gemäß der Beispiele 1 bis 4 verwendet werden

| | **Zahnpasta 1** % | **Zahnpasta 4** % |
|---|---|---|
| Wasser deion. | 30,60 | 31,60 |
| CMC | 1,20 | 1,20 |
| Solbrol M-Na | 0,20 | 0,20 |
| Saccharin Na | 0,10 | 0,10 |
| Titandioxid | 0,40 | 0,40 |
| Sorbitol 70 %ig | 40,00 | 40,00 |
| Kieselsäure gemäß Beispiel 1 | 25,00 | 22,00 |
| AEROSlL 200 | - | 2,00 |
| Aromaöl | 1,00 | 1,00 |
| Schaummittel | 1,50 | 1,50 |

| | **Zahnpasta 2** % | **Zahnpasta 5** % |
|---|---|---|
| Wasser deion. | 33,60 | 33,60 |
| CMC | 1,20 | 1,20 |
| Solbrol M-Na | 0,20 | 0,20 |
| Saccharin Na | 0,10 | 0,10 |
| Titandioxid | 0,40 | 0,40 |
| Sorbitol 70 %ig | 40,00 | 40,00 |
| Kieselsäure gemäß Beispiel 1 | 14,00 | - |
| Kieselsäure gemäß Beispiel 2 | - | 14,00 |
| Kieselsäure gemäß Beispiel 3 | - | 8,00 |
| Kieselsäure gemäß Beispiel 4 | 8,00 | - |
| Aromaöl | 1,00 | 1,00 |
| Schaummittel | 1,50 | 1,50 |

| | **Zahnpasta 3** % | **Zahnpasta 6** % |
|---|---|---|
| Wasser deion. | 39,60 | 39,60 |
| CMC | 1,20 | 1,20 |
| Solbrol M-Na | 0,20 | 0,20 |
| Saccharin Na | 0,10 | 0,10 |
| Titandioxid | 0,40 | 0,40 |
| Sorbitol 70 %ig | 40,00 | 40,00 |
| Kieselsäure gemäß Beispiel 1 | 5,00 | - |
| Kieselsäure gemäß Beispiel 2 | - | 5,00 |
| Kieselsäure gemäß Beispiel 3 | - | 11,00 |
| Kieselsäure gemäß Beispiel 4 | 11,00 | - |
| Aromaöl | 1,00 | 1,00 |
| Schaummittel | 1,50 | 1,50 |

| | **Zahnpasta 7** % | **Zahnpasta 10** % |
|---|---|---|
| Wasser deion. | 7,95 | 12,45 |
| CMC | 0,50 | 0,50 |
| Farbstoff 1 %ig | 0,50 | 0,50 |
| Solbrol M-Na | 0,15 | 0,15 |
| Saccharin- Na | 0,20 | 0,20 |
| Polyethylenglykol 400 | 3,50 | 3,50 |
| Sorbitol 70 %ig | 40,00 | 40,00 |
| Glyzerin | 20,00 | 20,00 |
| Kieselsäure gemäß Beispiel 1 | 25,00 | 18,00 |
| AEROSIL 200 | - | 2,50 |
| Aromaöl | 1,00 | 1,00 |
| Schaummittel | 1,20 | 1,20 |

| | **Zahnpasta 8** % | **Zahnpasta 11** % |
|---|---|---|
| Wasser deion. | 10,95 | 10,95 |
| CMC | 0,50 | 0,50 |
| Farbstoff 1 %ig | 0,50 | 0,50 |
| Solbrol M-Na | 0,15 | 0,15 |
| Saccharin- Na | 0,20 | 0,20 |
| Polyethylenglykol 400 | 3,50 | 3,50 |
| Sorbitol 70 %ig | 40,00 | 40,00 |
| Glyzerin | 20,00 | 20,00 |
| Kieselsäure gemäß Beispiel 1 | 14,00 | - |
| Kieselsäure gemäß Beispiel 2 | - | 14,00 |
| Kieselsäure gemäß Beispiel 3 | - | 8,00 |
| Kieselsäure gemäß Beispiel 4 | 8,00 | - |
| Aromaöl | 1,00 | 1,00 |
| Schaummittel | 1,20 | 1,20 |

| | **Zahnpasta 9** % | **Zahnpasta 12** % |
|---|---|---|
| Wasser deion. | 11,95 | 11,95 |
| CMC | 0,50 | 0,50 |
| Farbstoff 1 %ig | 0,50 | 0,50 |
| Solbrol M-Na | 0,15 | 0,15 |
| Saccharin- Na | 0,20 | 0,20 |
| Polyethylenglykol 400 | 3,50 | 3,50 |
| Sorbitol 70 %ig | 40,00 | 40,00 |
| Glyzerin | 25,00 | 25,00 |
| Kieselsäure gemäß Beispiel 1 | 5,00 | - |
| Kieselsäure gemäß Beispiel 2 | - | 5,00 |
| Kieselsäure gemäß Beispiel 3 | - | 11,00 |
| Kieselsäure gemäß Beispiel 4 | 11,00 | - |
| Aromaöl | 1,00 | 1,00 |
| Schaummittel | 1,20 | 1,20 |

### Bestimmung der Fluoridfreigabedynamik als Maß für die Depotwirkung und des PH-Wertes in Abhängigkeit vor der Zeit an der Fällungskieselsäure gemäß Beispiel 1

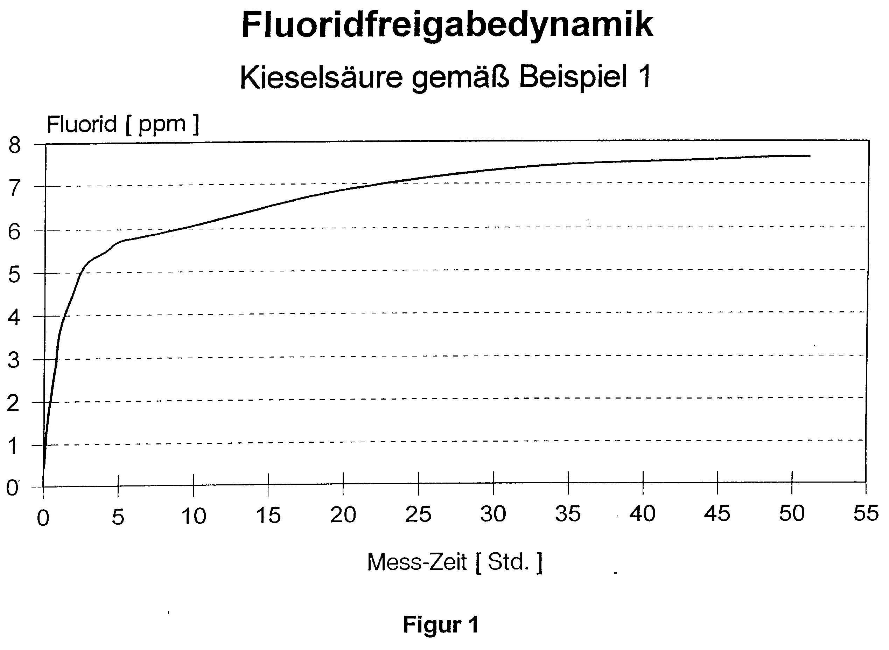

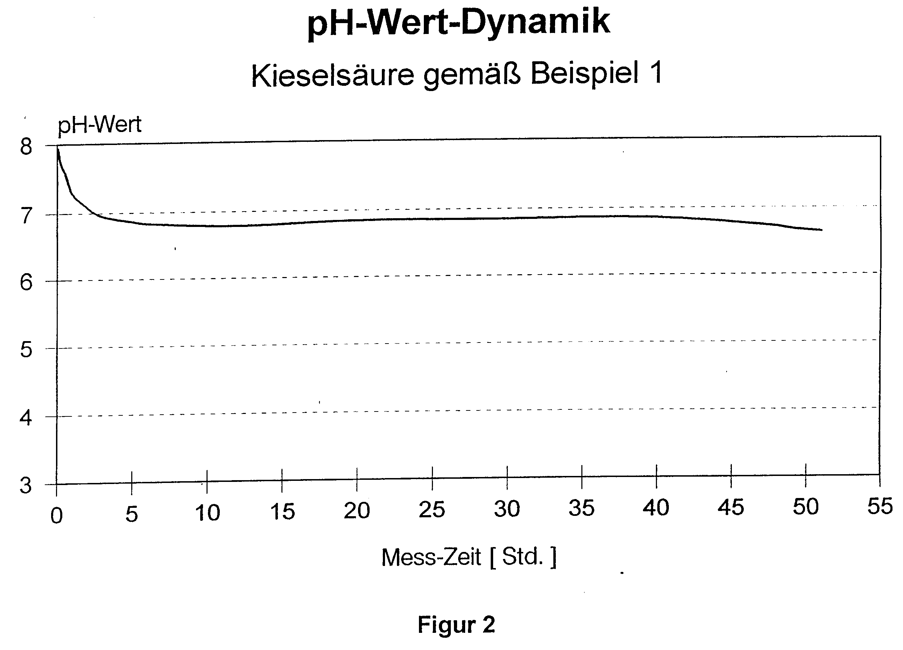

Siehe auch Figur 1 (Fluoridfreigabedynamik) und Figur 2 (pH-Wert-Dynamik).

| | **Menge an Fluorid bezogen auf die Fällungskieselsäure** | **pH-Wert** |
|---|---|---|
| nach 2 min. | 0,479 ppm | 7,95 |
| nach 5 min. | 0,767 ppm | 7,91 |
| nach 15 min. | 1,62 ppm | 7,71 |
| nach 30 min. | 2,24 ppm | 7,59 |
| nach 45 min. | 2,86 ppm | 7,42 |
| nach 60 min. | 3,62 ppm | 7,29 |
| nach 120 min. | 4,64 ppm | 7,09 |
| nach 150 min. | 5,08 ppm | 7,01 |
| nach 180 min. | 5,28 ppm | 6,96 |
| nach 240 min. | 5,48 ppm | 6,91 |
| nach 300 min. | 5,73 ppm | 6,87 |
| nach 360 min | 5,80 ppm | 6,84 |
| nach 24 Std. | 7,09 ppm | 6,86 |
| nach 25 Std. | 7,06 ppm | 6,79 |
| nach 26 Std. | 6,89 ppm | 6,75 |
| nach 48 Std. | 7,64 ppm | 6,73 |
| nach 49 Std. | 7,49 ppm | 6,69 |
| nach 50 Std. | 7,43 ppm | 6,67 |
| nach 51 Std. | 7,40 ppm | 6,65 |

Berechnung zur zuvor aufgeführten Tabelle:

| | | |
|---|---|---|
| Gesamt-Fluorid in der Prüfsuspension | [F] ges. | 50 ppm |
| Lösliches Fluorid | [F] löst. | 7,8 ppm |
| Freigesetztes Fluorid | [%] d. lösl. F⁻ | 97,9 |

Die erfindungsgemäße Kieselsäure gemäß Beispiel 1 besitzt ein Fluorid-Depot von 5 mg F'/g Kieselsäure, entsprechend 50 ppm in der Prüfsuspension. Aufgrund des Löslichkeitsprodukts von CaF₂ sind davon 7,8 ppm löslich. Das Experiment belegt, daß 97,9 % des löslichen Fluorids innerhalb von 2 Tagen abgegeben werden.

### Bestimmung des Gesamtfluoridgehaltes der Kieselsäure

Mit Hilfe einer Pyrohydrolyse und einer ionenchromatographischen Bestimmung wird der Gesamtfluoridgehalt der Kieselsäure bestimmt.

| **Kieselsäure** | **Meßergebnis [%]** |
|---|---|
| gemäß Beispiel 1 | 0,51 ± 0,02 |

Das Ergebnis zeigt, daß die theoretische Gesamtmenge des eingesetzten Fluorids von 5 mg F/g Fällungskieselsäure (0,5 Gew.-%) auf der Fällungskieselsäure fixiert wird.

### Putzversuche an Backenzähnen und deren Oberflächenanalyse mittels XPS/SIMS

An der erfindinngsgemäßen Fällungskieselsäure gemäß Beispiel 1 wird gezeigt, daß nach einem einmaligen Putzvorgang eine Einlagerung von Fluoridionen in die Zahnoberfläche erfolgt.

| **Parameter** | **Zahn-Nr. 1** | **Zahn-Nr. 2** | **Zahn-Nr. 3** |
|---|---|---|---|
| Polieren | Zahnoberfläche wird poliert | Zahnoberfläche wird poliert | Zahnoberfläche wird poliert |
| 1. Oberflächenanalyse (= Ausgangszustand vor dem Putzen) | Analyse mittels XPS / SIMS | Analyse mittels XPS / SIMS | Analyse mittels XPS / SIMS |
| Herstellung von Zahnpasten-Putzkörper-Suspensionen | Suspension Colgate-Zahnpaste Kariesschutz wird 1:1 in Wasser homogenisiert | Eine 20 % Kieselsäure gemäß Beispiel 1 enthaltende Standardpaste A wird 1:1 in Wasser dispergiert | Eine 20 % Kieselsäure gemäß Beispiel 2 und 1000 ppm CaF₂ enthaltende Standardpaste B wird 1 : 1 in Wasser dispergiert |
| Putzvorgang | 5 min. mit elektr. Zahnbürste auf einem Rütteltisch putzen | 5 min. mit elektr. Zahnbürste auf einem Rütteltisch putzen | 5 min. mit elektr. Zahnbürste auf einem Rütteltisch putzen |

### Röntgenphotoelektronenzpektrometrie (XPS)

Die XPS-Technik, die auf dem Prinzip des photoelektrischen Effektes beruht /1-4/, wird herangezogen, um die Oberflächenzusammensetzung der Zähne vor und nach der F-Behandlung zu bestimmen. Aufgrund der hohen Oberflächenspezifität der XPS können die Vorgänge in den obersten Nanometern, dem Grenzflächenbereich, in dem der Einbau von F bzw. der chemische Angriff auf den Zahnschmelz bzw. Abrasionsvorgänge erfolgen, gezielt analysiert werden.

Die Analyse erfaßt ausschließlich die obersten Atomlagen, also selektiv den Bereich in dem F-Einbau- bzw. Elutionsvorgänge stattfinden. Obwohl die Analyse nur wenige Atomlagen tief in das Material eindringt, wird der 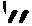 Analysenfleck" gezielt über den ganzen Zahn gesetzt, so daß der Einfluß von Mikroinhomogenitäten auf das Analysenergebnis ausgeschlossen wird, und eine für die ganze Zahnoberfläche relevante, makroskopische Information erhalten wird. Somit ist die Methode XPS um ca. den Faktor 1000 oberflächenempfindlicher, als die in der Elektronenmikroskopie eingesetzte Technik EDX (Energiedispersive Röntgenmikroanalyse).

Die XPS-Methode bietet den Vorteil der guten Quantifizierbarkeit. Insbesondere liefert sie auch Informationen über den chemischen Bindungszustand der Oberfläche von Zahnschmelz oder von Dentin. XPS ist daher ein in der Dentalforschung und an Zahnkliniken (insbesondere in den USA) international sehr gut etabliertes Meßverfahren, um z. B. auch die Komplexierung von Ca, das Ca/P-Verhältnis der Zahnoberfläche oder dem Dentin oder Effekte durch Cleanser und Primer zu verfolgen.

### Prinzip:

### 1. Einschleusen

Ein Backenzahn wird ohne weitere Vorbehandlung in die XPS-Versuchsanlage (Leybold LHS12) eingeschleust. Das verwendete Gerät besteht aus mehreren Vakuumkammern. In der ersten Kammer wird der Backenzahn vorsichtig von Raumluftbedingungen auf Vorvakuumbedingungen (ca. 10⁻2 mbar, ölfreies Drehschieberpumpenvakuum) gebracht, um Feuchtigkeit und andere z. T. organische, leicht desorbierbare Bestandteile insbesondere des Dentins abzupumpen. Danach erfolgt der Transfer des grob vorgetrockneten Zahnes in eine Präparationskammer. In dieser Kammer wird das Material auf Hoch- bzw. Ultrahochvakuumbedingungen (10⁻⁷-10⁻⁸ mbar, Turbopumpenvakuum) heruntergepumpt. Während dieser Zeit wird mit Hilfe eines Restgas-Quadrupolmassenspektrometers untersucht, welche flüchtigen Restkomponenten (insbesondere Wasser) unter diesen extremen Bedingungen noch aus dem Material entweichen. Die Abpumpvorgänge finden durchweg bei Raumtemperatur statt. Der Zahn wird somit nicht weiter belastet. Die solchermaßen konditionierte Probe wird schließlich in die eigentliche Analysenkammer (Basisdruck: 6 - 8 x 10⁻¹⁰ mbar, Turbopumpen-, Ionengetterpumpen- und Ti-Sublimationspumpenvakuum) überführt.

### 2. Meßvorgang

Die Zahnoberfläche wird unter Ultrahochvakuumbedingungen großflächig mit weicher Röntgenstrahlung beschossen (MgK_{*α*}-Strahlung, 1253.6 Elektronenvolt, Leistung 200 Watt). Hierdurch werden Photoemissionsprozesse ausgelöst. Es werden Elektronen freigesetzt (z. B. F1s, Ca2p, P2s, C1s, O1s etc.). Aufgrund der gewählten Anregungsenergie werden Phototelektronen ausgelöst, die nur eine sehr geringe mittlere freie Weglänge im Festkörper aufweisen. Somit ist die Methode für den Bereich der obersten Atomlagen spezifisch, d. h. im Falle von oxidischen Mineralien wie z. B. Hydroxylapatit/Fluorapatit werden die obersten 2 Nanometer selektiv erfaßt.

Nur die Elektronen, die unmittelbar an der Materialoberfläche ausgelöst wurden, können das Material verlassen und als Informationsträger dienen. Die kinetische Energie dieser emittierten Elektronen wird mit einem hemisphärischen Energieanalysator (CHA, Leybold EA11A) bestimmt. Aus der Bilanz zwischen gemessener Energie und der Energie der eingestrahlten Röntgenphotonen wird die Bindungsenergie der von den Oberflächenatomen des Zahnes abgelösten Photoelektronen gemessen. Trotz der hohen Oberflächenspezifität können ca. 1,5 cm² der Oberfläche mittels optischer Mikroskopie und eines Justierlasers einjustiert und integral analysiert werden. Der Einfluß von Mikroinhomogenitäten wird somit herausgemittelt.

### 3. Auswertung

### 1. Oberflächenkonzentrationen

Die gemessenen XPS-Spektren werden zunächst durch Polynomfits und Subtraktionsprogramme zur Entfernung sogenannter Röntgensatellitensignale behandelt. Danach werden alle mittels XPS nachgewiesenen Elemente (außer H und He) identifiziert. Die Quantifizierung erfolgt nach Untergrundsubtraktion gemäß Shirley /5/ unter Berücksichtigung der elementspezifischen relativen Empfindlichkeitsfaktoren.

### 2. Bindungszustände

Anhand der Bindungsenergiewerte kann zudem bestimmt werden, in welchem chemischen Bindungszustand die Elemente vorliegen. So kann z. B. zwischen Carbonat-Kohlenstoff und aliphatisch gebundenem Kohlenstoff auf Beton etc. unterschieden werden. Für eine mehr als qualitative Auswertung dieses wichtigen Aspektes ist es erforderlich, die probenspezifische elektrostatische Aufladung der schlecht elektrisch leitenden Materialien durch interne Referenzverfahren oder Kompensationstechniken zu berücksichtigen.

### Literatur:

/1/ K. Siegbahn et. al., Nova Acta Reg. Soc. Sci. Ups. Ser. IV Vol. 20 (1967)
/2/ Practical Surface Analysis, Ed. D. Briggs, M.P. Seah, Second Edition, 1990, John Wiley, Chichester und Salle + Sauerländer, Aarau
/3/ J.F. Moulder, W.F. Stickle, P.E. Sobol, K.D. Bomben, Handbook of X-ray Photoelectron Spectroscopy, Ed. J. Chastain, Perkin Elmer, Physical Electronics Division, Eden Prairie, Mi, USA, 1992
/4/ R. Holm, S. Storp, Methoden zur Untersuchung von Oberflächen, Ullmanns Enzyklopädie der technischen Chemie, Band 5, Verlag Chemie, Weinheim, 1980, S. 242 - 256
/5/ D.A. Shirley, Phys. Rev. B 5, 1992, 4709

### Rezeptur der verwendeteten Riefenwert Standardpasten

| **Rohstoff** | **Standardpaste A [%]** | **Standardpaste B [%]** |
|---|---|---|
| **1** dem. Wasser | 34,54 | 34,54 |
| **2** CMC, Blanose 7 MCF | 1,00 | 1.00 |
| **3** Konservierungsstoff Solbrol-M | 0,20 | 0,20 |
| 4 Süßstoff Saccharin | 0,10 | 0,10 |
| 5 CaF₂ | - | 0,21 |
| **6** Paraffinöl | 0,50 | 0,50 |
| **7** Sorbitol | 40,00 | 40.00 |
| **8** Aromaöl | 1,00 | 1,00 |
| Kieselsäure | 20 % Fällungskieselsäure gemäß Beispiel 1 | 20 % Fällungskieselsäure gemäß Beispiel 2 |

### Herstellung der Standardpasten A und B

Nach Anquellen des Bindemittels (CMC) in Wasser in der Retschmühle RM1 werden die Komponenten 3 bis 8 zugemischt und homogenisiert. Von der erhaltenen Mischung werden jeweils 160 g eingewogen und jeweils 40 g der Fällungskieselsäure gemäß den Beispielen 1 oder 2 bei laufender Retschmühle zugegeben. Nach vollständiger Einarbeitung der Fällungskieselsäuren werden die Pasten anschließend dreimal auf einem Exakt-Dreiwalzenstuhl homogenisiert.

### Herstellung der Zahnpasten-Putzkörper-Suspension

Anschließend werden die Standardpasten A und B sowie Colgate-Bifluorid jeweils 1 : 1 mit Wasser verdünnt und 5 min. mit einem Doppelflügelrührer bei 1500 U/min in einem 400 ml Becherglas dispergiert.

### Beschreibung des Putzvorgangs

Die kieselsäurehaltigen Zahnpastensuspensionen werden in eine Putzapparatur gefüllt und die Kaufläche polierter Humanzähne (Molare) 5 min. mit einer elektrischen Zahnbürste (Marke Broxodent) geputzt. Der für die Putzapparatur verwendete Rütteltisch wurde dabei auf 60 Schwingungen / min. eingestellt, um Sedimentbildung zu vermeiden. Nach dem Putzvorgang werden die Zähne unter fließendem fluoridfreiem Wasser gespült, getrocknet und oberflächenanalytisch untersucht.

Zu Vergleichsversuchen wird die handelsübliche Zahnpasta Colgate Bi-fluorid mituntersucht.

### Versuchsergebnisse

| | **Zahn 1 Colgate Bi-fluorid** | **Zahn 1 Colgate Bi-fluorid** | **Zahn 2 Standardpaste A** | **Zahn 2 Standardpaste A** | **Zahn 3 Standardpaste B** | **Zahn 3 Standardpaste B** |
|---|---|---|---|---|---|---|
| Zustand Humanzahn Kaufläche | poliert | geputzt | poliert | geputzt | poliert | geputzt |
| Flächenprozent Fluorid 1. Messung | 0,68 | 1,04 | 0,45 | 0,91 | 0,46 | 0,69 |
| Δ-Prozent 1. Messung | + 0,36 | | + 0,46 | | + 0,23 | |

| Wiederholungsmessung | | | | | | |
|---|---|---|---|---|---|---|
| Zustand Humanzahn Kaufläche | poliert | geputzt | poliert | geputzt | poliert | geputzt |
| Flächenprozent Fluorid 2. Messung | 0,33 | 0,75 | 0,41 | 0,91 | 0,35 | 0,33 |
| Δ-Prozent 2. Messung | + 0,42 | | + 0,50 | | -0,02 | |

Überraschenderweise konnte durch diese Ergebnisse gezeigt werden, daß die erfindungsgemäße Kieselsäure gemäß Beispiel 1 in Standardpaste A im Vergleich zu Standardpaste B, in der eine Abrasivkieselsäure und CaF₂ separat zugegeben wurde, und Colgate Bi-Fluorid, einer Zahnpaste mit propagierter Langzeitwirkung, enthaltend NaF, NaMFP und Calcium nach einmaligem Putzen eine signifikat höhere Fluorideinlagerung bewirkt. Dadurch ist zu erwarten, daß eine Verbesserung der Härteresistenz gegenüber Demineralisation gegeben ist. Gleichzeitig legt die unter 1 beschriebene Fluoridfreigabedynamik nahe, daß bei 37 °C in vivo eine verzögerte Abgabe des Fluorids über mehrere Tage erfolgt.

Die Zahnpaste Colgate mit Bi-Fluorid und Calzium ist im Handel erhältlich. Sie weist die folgenden Inhaltsstoffe (entspr. CTFA) auf:

Dicalcium Phosphate, Water, Glycerin, Sorbitol, Cellulose Gum, Sodium Laurylsulfate, Flavour, Tetra Sodium Pyrophosphate, Sodium Saccharin, Sodium Monofluorphosphate, Sodium Fluoride.

### Bestimmung der Fluoridfreigabedynamik von mit Alkali- und Erdalkalifluoriden dotierten Kieselsäuren

### 1.) Grundlagen

Mit Hilfe dieser Methode wird die Fluoridfreigabe fluorhaltiger Kieselsäuren in Abhängigkeit der Zeit untersucht. Damit läßt sich ermitteln, ob die Kieselsäure eine Depotwirkung besitzt oder nicht.

Die Messung erfolgt mittels ionenselektiver Elektrode und einem Ion-Analyzer. Zur Fluoriddotierung werden zum Beispiel Alkali- und Erdalkalisalze verwendet.

### 2.) Geräte und Reagenzien

### 2.1.) Geräte:

Analysenwaage Sartorius A 200 S
Spatellöffel 250 ml Poly-Schraubdeckelbehälter
Ika-Magnetrührer, ES 5, mit 2 cm Rührstab
Colora-Thermostat mit Wasserbad
ORION pH/mV und Ionenmeter Modell EA 940
mit pH-Elektrode
mit ionenselektiver Elektrode

### 2.2.) Reagenzien

Dest. Wasser
ORION Fluorid-Standardlösung 100 ppm zur Herstellung folgender Eichlösungen:
   100 ppm
   50 ppm
   20 ppm
pH-Pufferlösungen pH 4,0, 7,0 und 10,0

### 3.) Arbeitssicherheit:

NaF ist giftig
   R 23/24/25 S 1/2-26-44
Im sauren pH sich entwickeltendes HF ist sehr giftig
   R 26/27/28-35 S 2/9-26-36/37-45

### 4.) Durchführung

### 4.1.) Ansetzen der Fluorid-Kalibrierlösungen

Der vorliegende ORION-Fluoridstandard, 100 ppm, wird für die Kalibrierung 1:1 bzw. 1:5 mit destilliertem Wasser verdünnt und in 250 ml Poly-Schraubdeckelbehältern gelagert.

### 4.2.) Standardisierung

Die Kalibrierung des Ionenmeters ist vor Beginn jeder Meßreihe vorzunehmen. Hierzu werden die zuvor angegebenen Kalibrier- bzw. Pufferlösungen verwendet. Vor jedem Eintauchen der Elektrode in eine Kalibrierlösung wird die Elektrode mit dest. Wasser abgespült und vorsichtig abgetrocknet. Die Angabe des Geräteherstellers sind dabei zu beachten.

### 4.3.) Messungen

Es werden 100 g einer 1-prozentigen Kieselsäure/Wasser Suspension auf 10 mg genau in ein 250 ml Poly-Schraubdeckelbehälter eingewogen. Um den Kontakt mit eventuell entweichender Flußsäure zu verhindern, werden die Arbeiten im Abzug durchgeführt.

Die Suspension wird im Wasserbad auf 37 °C temperiert und mittels Magnetrührer bei Rührgeschwindigkeit 3 gerührt.

Die Messung der Fluoridfreigabe erfolgt mit Hilfe einer ionenselektiven Elektrode. Hierzu wird die Membran der ionenselektiven Elektrode mit der, in der Elektrode vorhandenen Füllösung nach Herstellerangabe gespült und der Füllstand erneut korrigiert. Die Elektrode wird mit destilliertem Wasser abgespült und mit einem weichen Tuch abgetrocknet, die Spitze der Elektrode wird in die Suspension eingetaucht und der Fluoridgehalt in bestimmten Zeitintervallen unter Rühren in ppm abgelesen und notiert. Die Meßintervalle werden von 2 min bis mehrere Stunden bis zur Konstanz des Meßwertes gesteigert.

Gleichzeitig wird der pH-Wert der Suspension gemessen. Zur Messung wird die Elektrode mit destilliertem Wasser abgespült und abgetrocknet. Der Ion-Analyzer wird nun auf die pH-Messung umgestellt. Die Elektrode wird in die Suspension eingetaucht und der pH-Wert unter Rühren gemessen und notiert.

Für weitere Messungen des Fluoridgehaltes und des pH-Wertes verbleiben die Elektroden in der Suspension; es erfolgt lediglich ein Umschalten auf die jeweilige Elektrodenart für die Erfassung der Meßwerte.

### 5.) Auswertung

Die Fluoridfreigabedynamik wird graphisch als Fluoridkonzentration als Funktion der Zeit dargestellt. Im Ergebnisprotokoll werden folgende Angaben gemacht:
1)abgelesene Fluoridkonzentration nach Erreichen des Gleichgewichtszustandes mit entsprechender Zeitangabe, [F]_{eq} in ppm
2)theoretische Fluoridkonzentration von 1 g KS / 100 g Suspension [F]_{ges.} in ppm
3) lösliche Fluoridkonzentration von 1 g KS / 100 g Suspension [F]_{lösl.} in ppm
4)Angabe des Prozentsatzes der erreichten Fluoridkonzentration [F]_{eq} von der löslichen Fluoridkonzentration [F]_{lösl.}

Die Ergebnisse zeigen, daß es möglich ist, eine fluoriddotierte Fällungskieselsäure herzustellen, die anwendungstechnisch der Referenzfällungskieselsäure ohne Fluorid entspricht, aber die gewünschte Deptowirkung aufweist. Weitere Beispiele zeigen, daß Abrasivkieselsäuren, Verdickungskieselsäuren und bifunktionelle Kieselsäuren als Wirkstoffträger gemäß der Erfindung verwendet werden können.

Stellvertretend dafür soll Beispiel 3 genannt werden, das nach Variante 4 belegt wird und deren Depotwirkung ebenfalls nachgewiesen wird.

## Patentansprüche

1. Wirkstoff enthaltende Fällungskieselsäure, gekennzeichnet durch eine charakteristische, verzögerte Wirkstofffreigabe.

2. Verfahren zur Herstellung der Wirkstoff enthaltenden Fällungskieselsäure gemäß Anspruch 1, dadurch gekennzeichnet, daß man eine Fällungskieselsäure auf bekanntem Wege herstellt, wobei ein in Wasser mäßig bis schwer löslicher Wirkstoff entweder in die Fällungsvorlage oder in die Fällungssuspension gegeben oder zusammen mit dem gewaschenen und gegebenenfalls redispergierten Fällungskieselsäureteig getrocknet oder gleichzeitig mit dem getrockneten Material vermahlen wird.

3. Verwendung der Wirkstoff enthaltenden Fällungskieselsäure gemäß Anspruch 1 in Mundpflegemitteln.

4. Verwendung der Wirkstoff enthaltenden Fallungskieselsäure gemäß Anspruch 3 als Fluoid-Ionen abgebenden Zusatz zu Mundpflegemitteln.
